# EUROPEAN PATENT SPECIFICATION

(11) **EP 2 303 842 B1**
(45) Date of publication and mention of the grant of the patent: **31.10.2012**
(21) Application number: 09772245.8
(22) Date of filing: 27.05.2009
(51) Int. Cl.: C07D 211/90, A61K 31/4422, A61P 9/00

(54) **A process for producing lercanidipine.HCl form V**
Verfahren zur Herstellung von Lercanidipin.HCl Form V
Procédé pour la fabrication de la forme V de la lercanidipine.HCl

(30) Priority: 02.07.2008 EP 08159540
(43) Date of publication of application: 06.04.2011
(73) Proprietor: Recordati Ireland Limited, Ringaskiddy County Cork (IE)
(72) Inventor: LEONARDI, Amedeo, I-20154 Milano (IT); MOTTA, Gianni, I-20030 Barlassina (MI) (IT); JACQUET, Luc, I-21047 Saronno (VA) (IT)
(74) Representative: Pistolesi, Roberto
(86) International application number: PCT/EP2009/056439
(87) International publication number: WO 2010/000545

(56) References cited:
- WO-A-2007/031865

## Description

### A process for producing Lercanidipine.HCl Form V

The present invention relates to a new process for obtaining crystalline Lercanidipine.HCl Form V. The process includes (i) crystallization from a mixture of iPrOAc and an aprotic polar organic solvent, such as ACN, Me₂CO, DMF or DMA or (ii) slurrying Lercanidipine.HCl in iPrOAc.

### Glossary of technical terms

- HPLC: High Performance Liquid Chromatography
- DSC: Differential Scanning Calorimetry
- XRPD: X-Ray Powder Diffraction
- NMR: Nuclear Magnetic Resonance
- DMF: Dimethylformamide
- DMA: Dimethylacetamide
- iPrOAc: Isopropyl acetate
- Me₂CO: Acetone
- EtOH: Ethanol
- MeOH: Methanol
- ACN: Acetonitrile
- RT: Room Temperature (i.e. preferably from 15 to 30° C, more preferably from 20 to 25° C)
- FI: Lercanidipine.HCl Form I
- FII: Lercanidipine.HCl Form II
- FV: Lercanidipine.HCl Form V
- T: Temperature

### Background of the invention

Lercanidipine (methyl 1,1,N-trimethyl-N-(3,3-diphenylpropyl)-2-aminoethyl 1,4-dihydro-2,6-dimethyl-4-(3-nitrophenyl)pyridine-3,5-dicarboxylate) is a highly lipophitic dihydropyridine calcium antagonist with a long duration of action and high vascular selectivity. Lercanidipine's biological activity derives from its ability to competitively antagonize the dihydropyridine subunit of the L-type calcium channel.

Lercanidipine is useful as an anti-hypertensive. Lercanidipine lowers blood pressure by blocking calcium channels of arterial smooth muscle, thus decreasing peripheral vascular resistance. Lercanidipine produces no negative cardiac inotropism and only occasional mild reflex tachycardia, which is generally of short duration. Lercanidipine has been approved for the treatment of hypertension and has been marketed far-back in several European countries under the trademark Zanidip^{™}.

The hydrochloride salt of Lercanidipine is commercially available from Recordati S.p.A. (Milan, Italy). Methods of preparing Lercanidipine.HCl, as well as methods of resolving Lercanidipine into individual enantiomers are described in US 4705797, US 5767136, US 4968832, US 5912351, US 5696139, US 2003/0069285 and US 2003/0083355.

WO 2006/046830 discloses amorphous Lercanidipine with improved solubility and bioavailability and methods for preparing the same.

WO 2006/089787, discloses amorphous Lercanidipine.HCl and methods for preparing the same.

US 6852737 discloses crystalline Lercanidipine.HCl forms I and II and processes for their preparations

WO2007/031865 discloses crystalline Lercanidipine.HCl Form V and process for the preparation thereof; more in details, crystalline Lercanidipine.HCl Form V is obtained by recrystallization from MeOH/iPrOAc.

### Description of the invention

The object of the present invention is a process for obtaining crystalline Lercanidipine.HCl Form V other than that disclosed in WO2007/031865, namely recrystallization from MeOH/iPrOAc.

For this purpose, solubility determination at RT of FI, FII and FV in water, HCl 0.1 M and some organic solvents has been carried out as described in the experimental section.

A further screening has been done keeping iPrOAc as antisolvent and using aprotic polar organic solvents like DMF, DMA, ACN and acetone. The results of such a screening are summarized in Table 1.

**Table 1**

| Ex | Solvents | T | Result | Residual Solvents (% molar) |
|---|---|---|---|---|
| I | DMF | RT | FV | 5.3% iPrOAc |
| 1 | | 0°C | FV | 3.3% iPrOAc |
| 2 | DMA | RT | FV | 2% iPrOAc |
| 3 | ACN | RT | FV | 4% iPrOAc |
| 3 | | 0°C | FV | 4% iPrOAc |
| 4 | acetone | RT | FV | 13% iPrOAc |
| 4 | | DOC | FV | 3% iPrOAc |

As it shall be appreciated, at least in small scale, formation of Form V seems to be surprisingly favoured by working at RT. As it shall be appreciated from the experimental section, Form V has also been obtained by slurrying Lercanidipine.HCl in iPrOAc at RT. The term "slurrying" has the same meaning as normally understood by a skilled practitioner of this field, that is keeping a given product (in this case preferably amorphous Lercanidipine.HCl) under stirring in a solvent in which such a product is not or very poorly soluble. The object of the present invention is thus represented by a process for obtaining crystalline Lercanidipine.HCl Form V, which comprises: slurrying Lercanidipine.HCl in iPrOAc.

According to another embodiment, the mixture obtained upon slurrying with iPrOAc is kept under stirring, preferably for 12 to 36 hours, more preferably for 20 to 28 hours; the stirring temperature is normally of -5 to 30° C, preferably of 15 to 30° C, more preferably of 20 to 25° C.

According to a preferred embodiment, from 5 to 50 liters of iPrOAc are used per mole of Lercanidipine.HCl; preferably from 6.5 to 32 liters of iPrOAc.

According to another preferred embodiment, a mole of Lercanidipine.HCl is slurried in from 5 to 10 liters of iPrOAc; preferable from 6 to 7 liters of iPrOAc.

From preliminary evidences it appears that the crystalline Lercanidipine.HCl Form V obtainable according to the process of the present invention may have improved properties with respect to that obtained according to the process disclosed in WO2007/031865.

The examples reported in the experimental section are illustrative in nature of the various aspects of the present invention.

### Experimental section

### Example 1 - Slurry of amorphous Lercanidipine.HCl in iPrOAc

2mL of a 100 mg/mL slurry of amorphous Lercanidipine.HCl was left for 24h under magnetic stirring at RT. The resulting solid was collected by suction and dried in an oven at 50°C for about 48 hours.

### Example 2 - Solubility

Suspensions in different solvents, ranging in concentration from 20 mg/mL to 400 mg/mL, depending on the solubility, were shaken with thermomixer at 25°C, rpm=9000 for 24h. An aliquot was taken, centrifuged for 5 minutes at 13000 rpm, the supernatant was transferred in HPLC vials, opportunely diluted and assayed. For aqueous solvent the supernatant was further filtered with a 0.2 µm PVDF filter and centrifuged for 5 minutes at 8000 rpm.

Solubility data (mg/mL) obtained are gathered in Table 2.

**Table 2: solubility**

| Solvent | FI | FII | FV |
|---|---|---|---|
| H₂O | 1.6 | 0.30 | 0.96 |
| HCl 0.1M | 0.20 | 0.009 | 0.07 |
| MeOH | 263.9 | 83.9 | 221.9 |
| EtOH | 28.2 | 10.5 | 46.72 |
| ACN | 13.8 | 4.8 | 26.1 |
| acetone | 9.4 | 2.7 | 18.5 |

### Instrumentation

### DSC

Mettler Thermograms: 10 mg of material is analyzed in an open aluminium pan, using a Mettler DSC20 instrument with a temperature ramp of 10°C/min from 25°C to 250°C, except as otherwise mentioned.

Perkin Elmer Thermograms: 2 mg of material is analyzed in open Aluminium pan, using a Mettler Diamond instrument with a temperature ramp of 10°C/min from 25°C to 250°C, except as otherwise mentioned.

### HPLC

HPLC-DAD: HP 1050 separation module controlled by HP Chem-station.

**Method:**

| | | | |
|---|---|---|---|
| Column | Xterra RP 18 5 µm 4.6x50 mm | | |
| Temperature | Room temperature | | |
| Mobile phase | A: Acetonitrile | | |
| | B: NH₄HCO₃ 20 mM pH 8 | | |
| Flow rate | 1.8 mL/min | | |
| Gradient | TIME (min.) | %A | %B |
| | 0 | 10 | 90 |
| | 4 | 100 | 0 |
| | 4.5 | 100 | 0 |
| | 4.6 | 10 | 90 |
| | 6 | 10 | 90 |
| Detector | λ: 280 nm | | |

Standard and calibration: automatic three point calibration (0.5 mg/mL, 1.0 mg/mL, 1.5 mg/mL), including the zero, using HP Chem-station program.

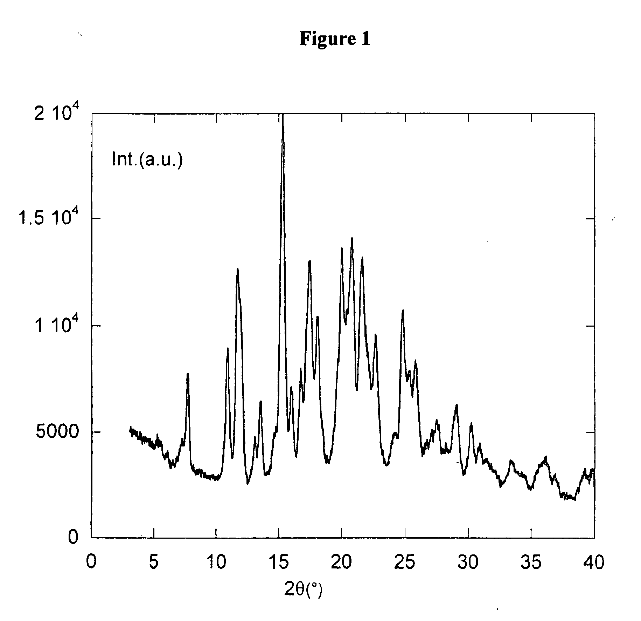

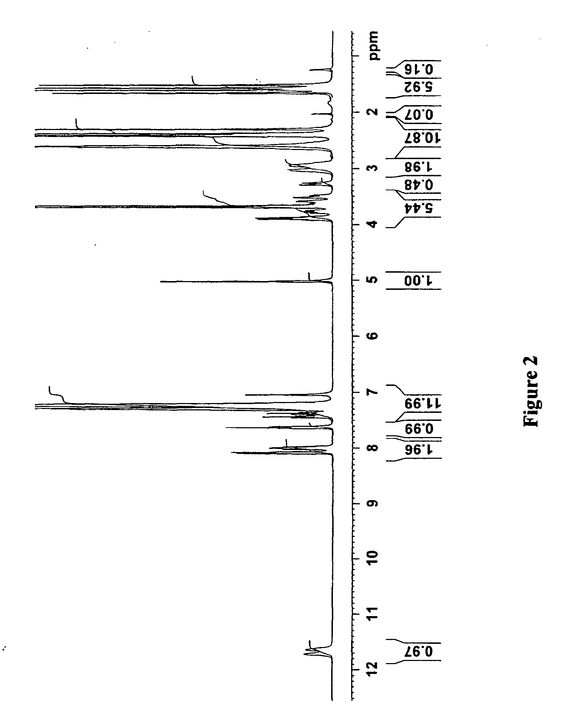

## Claims

1. A process for obtaining crystalline Lercanidipine.HCl Form V which comprises slurrying Lercanidipine.HCl in iPrOAc.

2. The process according to claim 1, **characterized in that** amorphous Lercanidipine.HCl is slurried in iPrOAc.

3. The process according to claim 1, **characterized in that** the mixture obtained upon mixing with iPrOAc is kept under stirring.

4. The process according to claim 3, **characterized in that** stirring is done for 12 to 36 hours.

5. The process according to claim 4, **characterized in that** stirring is done for 20 to 28 hours.

6. The process according to claim 3, **characterized in that** stirring is done at -5 to 30° C.

7. The process according to claim 6, **characterized in that** stirring is done at 15 to 30° C.

8. The process according to claim 7, **characterized in that** stirring is done at 20 to 25° C.

9. The process according to claim 1, **characterized in that** a mole of Lercanidipine.HCl is slurried in from 5 to 10 liters of iPrOAc.

10. The process according to claim 9, **characterized in that** a mole of Lercanidipine.HCl is slurried in 6 to 7 liters of iPrOAc.

## Patentansprüche

1. Verfahren zum Erhalt von kristallinem Lercanidipin-HCl Form V, umfassend das Aufschlämmen von Lercanidipin-HCl in iPrOAc.

2. Verfahren gemäss Anspruch 1, **dadurch gekennzeichnet, dass** amorphes Lercanidipin-HCl in iPrOAc aufgeschlämmt wird.

3. Verfahren gemäss Anspruch 1, **dadurch gekennzeichnet, dass** die nach dem Mischen mit iPrOAc erhaltene Mischung weitergerührt wird.

4. Verfahren gemäß Anspruch 3, **dadurch gekennzeichnet, dass** für 12 bis 36 Stunden gerührt wird.

5. Verfahren gemäss Anspruch 4, **dadurch gekennzeichnet, dass** für 20 bis 28 Stunden gerührt wird.

6. Verfahren gemäss Anspruch 3, **dadurch gekennzeichnet, dass** bei -5 bis 30°C gerührt wird.

7. Verfahren gemäss Anspruch 6, **dadurch gekennzeichnet, dass** bei 15 bis 30°C gerührt wird.

8. Verfahren gemäss Anspruch 7, **dadurch gekennzeichnet, dass** bei 20 bis 25°C gerührt wird.

9. Verfahren gemäss Anspruch 1, **dadurch gekennzeichnet, dass** 1 mol Lercanidipin-HCl in 5 bis 10 ℓ iPrOAc aufgeschlämmt wird.

10. Verfahren gemäss Anspruch 9, **dadurch gekennzeichnet, dass** 1 mol Lercanidipin-HCl in 6 bis 7 ℓ iPrOAc aufgeschlämmt wird.

## Revendications

1. Procédé pour obtenir la forme V cristalline du chlorhydrate de lercanidipine (lercanidipine.HCl), qui consiste à mettre en suspension du chlorhydrate de lercanidipine dans de l'iPrOAc.

2. Procédé selon la revendication 1, **caractérisé en ce que** du chlorhydrate de lercanidipine amorphe est mis en suspension dans de l'iPrOAc.

3. Procédé selon la revendication 1, **caractérisé en ce que** le mélange obtenu suite au mésange avec l'iPrOAc est maintenu sous agitation.

4. Procédé selon à revendication 3, **caractérisé en ce que** l'agitation est réalisée pendant 12 à 36 heures.

5. Procédé selon la revendication 4, **caractérisé en ce que** l'agitation est réalisée pendant 20 à 28 heures.

6. Procédé selon la revendication 3, **caractérisé en ce que** l'agitation est réalisée entre -5 et 30 °C.

7. Procédé selon la revendication 6, **caractérisé en ce que** l'agitation est réalisée entre 15 et 30 °C.

8. Procédé selon la revendication 7, **caractérisé en ce que** l'agitation est réalisée entre 20 et 25 °C.

9. Procédé selon la revendication 1, **caractérisé en ce qu'**une mole de chlorhydrate de lercanidipine est mise en suspension dans 5 à 10 litres d'iPrOAc.

10. Procédé selon la revendication 9, **caractérisé en ce qu'**une mole de chlorhydrate de lercanidipine est mise en suspension dans 6 à 7 litres d'iPrOAc.
